# EUROPEAN PATENT APPLICATION

(11) **EP 4 769 426 A1**
(43) Date of publication of application: **01.07.2026**
(21) Application number: 25227908.8
(22) Date of filing: 31.12.2025
(51) Int. Cl.: G16H 30/20, G16H 50/20, G16H 30/40

(54) **SYSTEMS AND METHODS FOR MEDICAL SCANNING**

(30) Priority: 31.12.2024 CN 202412000039
(71) Applicant: Shanghai United Imaging Healthcare Co., Ltd., Shanghai 201807 (CN)
(72) Inventor: FU, Lei, Shanghai, 201807 (CN); MENG, Xiaolin, Shanghai, 201807 (CN)
(74) Representative: Wang, Bo

(57) **Abstract**

A method for medical scanning is provided. The method includes: obtaining, via a user terminal, an input of a user specifying scan requirements with respect to a target subject to be scanned; extracting, from the input, scan requirement information; obtaining medical record information of the target subject; and generating a scanning scheme for the target subject by processing the scan requirement information and the medical record information, wherein the processing of the scan requirement information and the medical record information is performed with reference to long-term memory information stored in the at least one storage device, and the long-term memory information comprises at least one of a rule database, a knowledge graph, or a historical scanning scheme database.

## Description

### TECHNICAL FIELD

The present disclosure relates to the field of medical scanning, and in particular to systems and methods for determining a scanning scheme.

### BACKGROUND

In the field of medical diagnostics, patients are often scanned using medical scanning devices, with subsequent diagnosis based on the acquired images. However, in practice, the complexity of operating such devices results in a heavy reliance on the operator's expertise. This can lead to low scanning efficiency and significant variability in image quality across different institutions. To address this issue, standardized workflows based on predefined scanning schemes are often implemented to simplify operations. Nevertheless, these schemes lack sufficient flexibility and struggle to meet the diverse and complex demands of clinical practice.

Therefore, there is a need for systems and methods for medical scanning that enables the automatic and precise generation of scanning schemes, thereby improving both scanning efficiency and image quality.

### SUMMARY

One or more embodiments of the present disclosure provide a method for medical scanning, the method is implemented on a computing device having at least one processor and at least one storage device, the method includes: obtaining, via a user terminal, an input of a user specifying scan requirements with respect to a target subject to be scanned; extracting, from the input, scan requirement information; obtaining medical record information of the target subject; and generating a scanning scheme for the target subject by processing the scan requirement information and the medical record information, wherein the processing of the scan requirement information and the medical record information is performed with reference to long-term memory information stored in the at least one storage device, and the long-term memory information comprises at least one of a rule database, a knowledge graph, or a historical scanning scheme database.

One or more embodiments of the present disclosure provide a system, the system includes: a user terminal; at least one storage device configured to store long-term memory information, the long-term memory information comprises at least one of a rule database, a knowledge graph, or a historical scanning scheme database; an intelligent engine communicatively communicated with the user terminal and the at least one storage device, configured to: obtain, via the user terminal, an input of a user specifying scan requirements with respect to a target subject to be scanned; extract, from the input, scan requirement information; obtain medical record information of the target subject; and generate a scanning scheme for the target subject by processing the scan requirement information and the medical record information, wherein the processing of the scan requirement information and the medical record information is performed with reference to the long-term memory information stored in the at least one storage device.

One or more embodiments of the present disclosure provide a computer-readable storage medium storing computer instructions, when a computer reads the computer instructions in the storage medium, the computer implements: obtaining, via a user terminal, an input of a user specifying scan requirements with respect to a target subject to be scanned; extracting, from the input, scan requirement information; obtaining medical record information of the target subject; and generating a scanning scheme for the target subject by processing the scan requirement information and the medical record information, wherein the processing of the scan requirement information and the medical record information is performed with reference to long-term memory information stored in the at least one storage device, and the long-term memory information comprises at least one of a rule database, a knowledge graph, or a historical scanning scheme database.

In some embodiments, the generating a scanning scheme for the target subject by processing the scan requirement information and the medical record information comprises: obtaining, via one or more sensing devices at a scanning site, on-site sensing information relating to the target subject; and generating the scanning scheme by processing the scan requirement information, the medical record information, and the on-site sensing information.

In some embodiments, the input includes a natural language input, and the natural language input is obtained by: presenting, via the user terminal, a digital avatar of an intelligent engine; and receiving the natural language input of the user during a natural language interaction with the digital avatar.

In some embodiments, the processing the scan requirement information and the medical record information comprises: determining, based on the scan requirement information and the medical record information, whether the target subject satisfies a preset condition; and in response to determining that the target subject satisfies the preset condition, generating the scanning scheme by querying the knowledge graph based on the scan requirement information and the medical record information.

In some embodiments, the generating the scanning scheme by querying the knowledge graph based on the scan requirement information and the medical record information comprises: extracting query entities from the scan requirement information and the medical record information, the query entities including one or more main query entities and one or more auxiliary query entities; constructing a main query condition based on the one or more main query entities and constructing an auxiliary query condition based on the one or more auxiliary query entities, and generating the scanning scheme by querying the knowledge graph using the main query condition and the auxiliary query condition.

In some embodiments, the processing the scan requirement information and the medical record information comprises: determining, based on the scan requirement information and the medical record information, whether the target subject satisfies a preset condition; and in response to determining that the target subject does not satisfy the preset condition, generating the scanning scheme by querying the historical scanning scheme database based on the scan requirement information and the medical record information.

In some embodiments, the historical scanning scheme database is queried using a retrieval-augmented generation (RAG) model.

In some embodiments, the historical scanning scheme database includes historical scanning schemes and their respective basis information vectors and evaluation information, the historical scanning schemes are ranked according to their evaluation information in the historical scanning scheme database, and the generating the scanning scheme by querying the historical scanning scheme database comprises: constructing a query vector based on the medical record information and the scan requirement information; determining candidate scanning schemes from the historical scanning schemes based on the query vector and the basis information vectors of the historical scanning schemes; determining a candidate scanning scheme with the highest rank among the candidate scanning schemes as the scanning scheme.

In some embodiments, the processing the scan requirement information and the medical record information comprises: determining a first candidate scanning scheme and its first confidence score by querying the rule database based on the scan requirement information and the medical record information; in response to determining that the first confidence score is lower than a first threshold, determining a second candidate scanning scheme and its second confidence score by querying the knowledge graph based on the scan requirement information and the medical record information; in response to determining that the second confidence score is lower than a second threshold, determining the scanning scheme by querying the historical scanning scheme database based on the scan requirement information and the medical record information.

In some embodiments, the scan requirement information and the medical record information comprises: generating at least two candidate scanning schemes by respectively querying the rule database, the knowledge graph, and the historical scanning scheme database based on the scan requirement information and the medical record information; for each of the at least two candidate scanning schemes, determining a confidence score of the candidate scanning scheme by processing the scan requirement information, the medical record information, and the candidate scanning scheme using a confidence evaluation model; and determining the scanning scheme based on the at least two candidate scanning schemes and their confidence scores.

In some embodiments, the determining the scanning scheme based on the at least two candidate scanning schemes and their confidence scores comprises: displaying, via the user terminal, the at least two candidate scanning schemes, their respective confidence scores, and their respective decision rationale; receiving, via the user terminal, a user selection input by the user; and determining the scanning scheme from the at least two candidate scanning schemes based on the user selection.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities and combinations set forth in the detailed examples discussed below.

### BRIEF DESCRIPTION OF THE DRAWINGS

The present disclosure is further described by way of exemplary embodiments. These exemplary embodiments are described in detail with reference to the accompanying drawings. These embodiments are nonlimiting. In these embodiments, the same numerals refer to the same structures, wherein:
FIG. 1 is a schematic diagram illustrating a medical scanning system according to some embodiments of the present disclosure;
FIG. 2 is a schematic diagram illustrating a computing device according to some embodiments of the present disclosure;
FIG. 3 is a flowchart illustrating an exemplary process of medical scanning according to some embodiments of the present disclosure;
FIG. 4 is a flowchart illustrating an exemplary process of generating a scanning scheme based on scan requirement information and medical record information according to some embodiments of the present disclosure;
FIG. 5 is a schematic diagram illustrating an exemplary process of generating a scanning scheme by querying a knowledge graph according to some embodiments of the present disclosure;
FIG. 6 is a schematic diagram illustrating an exemplary process of generating a scanning scheme by querying a historical scanning scheme database according to some embodiments of the present disclosure;
FIG. 7 is a schematic diagram illustrating another exemplary process of generating a scanning scheme based on scan requirement information and medical record information according to some embodiments of the present disclosure;
FIG. 8 is another schematic diagram illustrating a process of generating a scanning scheme based on scan requirement information and medical record information according to some embodiments of the present disclosure;
FIG. 9 is a block diagram of an intelligent engine according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that "system," "device," "unit," and/or "module" as used herein is a manner used to distinguish different components, elements, parts, sections, or assemblies at different levels. However, if other words serve the same purpose, the words may be replaced by other expressions.

As shown in the present disclosure and claims, the words "one," "a," "a kind," and/or "the" are not especially singular but may include the plural unless the context expressly suggests otherwise. In general, the terms "comprise," "comprises," "comprising," "include," "includes," and/or "including," merely prompt to include operations and elements that have been clearly identified, and these operations and elements do not constitute an exclusive listing. The methods or devices may also include other operations or elements.

It will be understood that, although the terms "first," "second," "third," "fourth," etc., may be used herein to describe various elements, these elements should not be limited by these terms. These terms are only used to distinguish one element from another. For example, a first element could be termed a second element, and, similarly, a second element could be termed a first element, without departing from the scope of example embodiments of the present invention.

The flowcharts used in the present disclosure illustrate operations that systems implement according to some embodiments of the present disclosure. It should be understood that the previous or subsequent operations may not be accurately implemented in order. Instead, each step may be processed in reverse order or simultaneously. Meanwhile, other operations may also be added to these processes, or a certain step or several steps may be removed from these processes.

In the present disclosure, the subject may include a biological object and/or a non-biological object. The biological object may be a human being, an animal, a plant, or a specific portion, organ, and/or tissue thereof. For example, the subject may include the head, the neck, the thorax, the heart, the stomach, a blood vessel, a soft tissue, a tumor, a nodule, or the like, or any combination thereof. In some embodiments, the subject may be a man-made composition of organic and/or inorganic matters that are with or without life. The terms "object" and "subject" are used interchangeably in the present disclosure.

In the present disclosure, the term "image" may refer to a two-dimensional (2D) image, a three-dimensional (3D) image, or a four-dimensional (4D) image (e.g., a time series of 3D images). In some embodiments, the term "image" may refer to an image of a region (e.g., a region of interest (ROI)) of a subject. In some embodiments, the image may be a medical image, an optical image, etc.

In the present disclosure, a representation of a subject (e.g., an object, a patient, or a portion thereof) in an image may be referred to as "subject" for brevity. For instance, a representation of an organ, tissue (e.g., a heart, a liver, a lung), or an ROI in an image may be referred to as the organ, tissue, or ROI, for brevity. Further, an image including a representation of a subject, or a portion thereof, may be referred to as an image of the subject, or a portion thereof, or an image including the subject, or a portion thereof, for brevity. Still further, an operation performed on a representation of a subject, or a portion thereof, in an image may be referred to as an operation performed on the subject, or a portion thereof, for brevity. For instance, a segmentation of a portion of an image including a representation of an ROI from the image may be referred to as a segmentation of the ROI for brevity.

For illustration purposes, the following description is provided to help better understand a medical scanning process. It is understood that this is not intended to limit the scope of the present disclosure. For persons having ordinary skills in the art, a certain amount of variations, changes, and/or modifications may be deducted under the guidance of the present disclosure. Those variations, changes and/or modifications do not depart from the scope of the present disclosure.

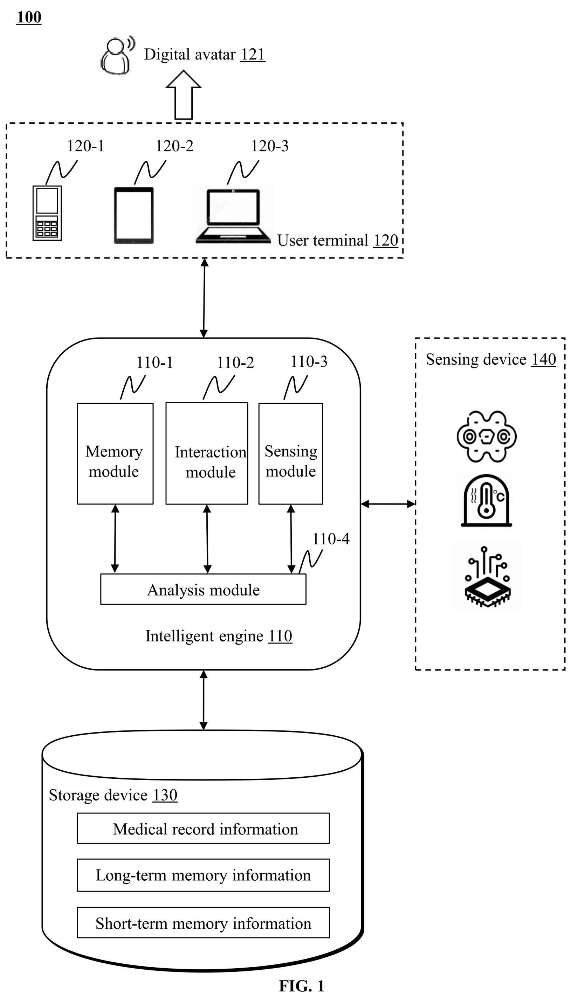

FIG. 1 is a schematic diagram illustrating a medical scanning system according to some embodiments of the present disclosure. As shown in FIG. 1, a medical scanning system 100 may include an intelligent engine 110, a user terminal 120, a storage device 130, and a sensing device 140. The count of each of the user terminal 120, the storage device 130, the sensing device 140 can be one or more. Components in the medical scanning system 100 may be communicatively connected. For example, as shown in FIG. 1, the intelligent engine 110 may be communicatively connected with the user terminal 120, the storage device 130, and the sensing device 140, respectively.

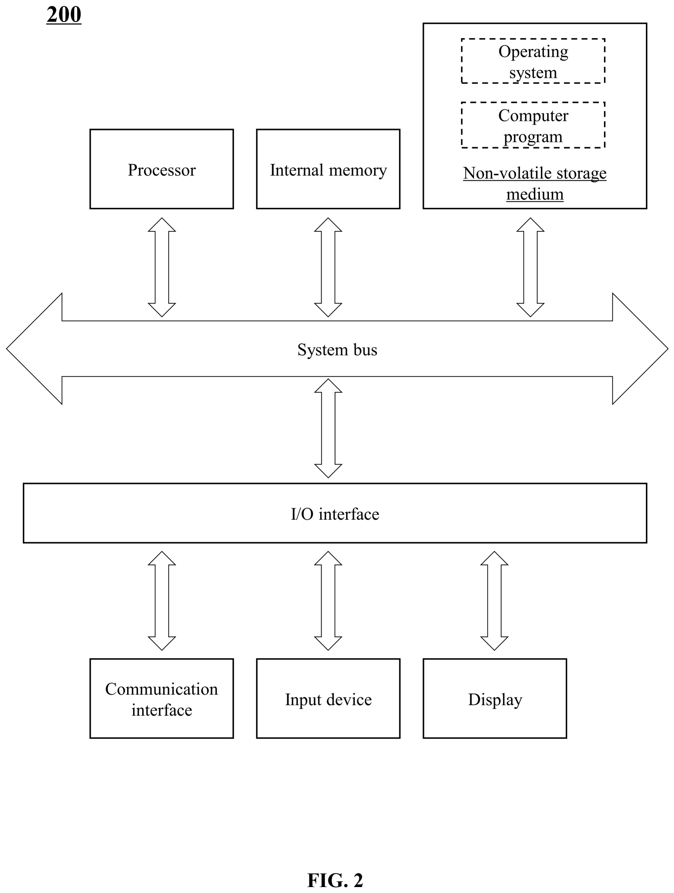

The intelligent engine 110 refers to a core computing and decision-making unit of the medical scanning system 100. The intelligent engine 110 is configured to fuse, understand, and reason multi-source information to automatically generate a personalized scanning scheme for a target subject. In some embodiments, the intelligent engine 110 may be implemented on at least one processor. In some embodiments, the processor(s) may be a single server or a server group. The server group may be centralized or distributed. In some embodiments, the processor(s) may be local or remote. In some embodiments, the processor(s) may be implemented on a cloud platform. Merely by way of example, the cloud platform may include a private cloud, a public cloud, a hybrid cloud, a community cloud, a distributed cloud, an inter-cloud, a multi-cloud, or the like, or any combination thereof. In some embodiments, the intelligent engine 110 may be implemented on a computing device. The computing device includes at least one processor and at least one storage device. The at least one storage device may include a built-in storage device and/or an external storage device of the computing device. In some embodiments, the intelligent engine 110 may be implemented by the computing device as shown in FIG. 2.

As shown in FIG. 1, the intelligent engine 110 may include a sensing module 110-3, an interaction module 110-2, a memory module 110-1, and an analysis module 110-4. The user terminal 120 is communicatively connected with the interaction module 110-2. The sensing module 110-3 is communicatively connected with the sensing device 140. The memory module 110-1 is communicatively connected with the storage device 130. The analysis module 110-4 is communicatively connected with the sensing module 110-3, the interaction module 110-2, and the memory module 110-1, respectively.

The sensing module 110-3 is communicatively connected with the sensing device 140, configured to receive on-site sensing information collected by the sensing device 140. The sensing module 110-3 is also configured to send related instructions (e.g., start or stop instructions, data transmission instructions, etc.) to the sensing device 140 to control the sensing device 140.

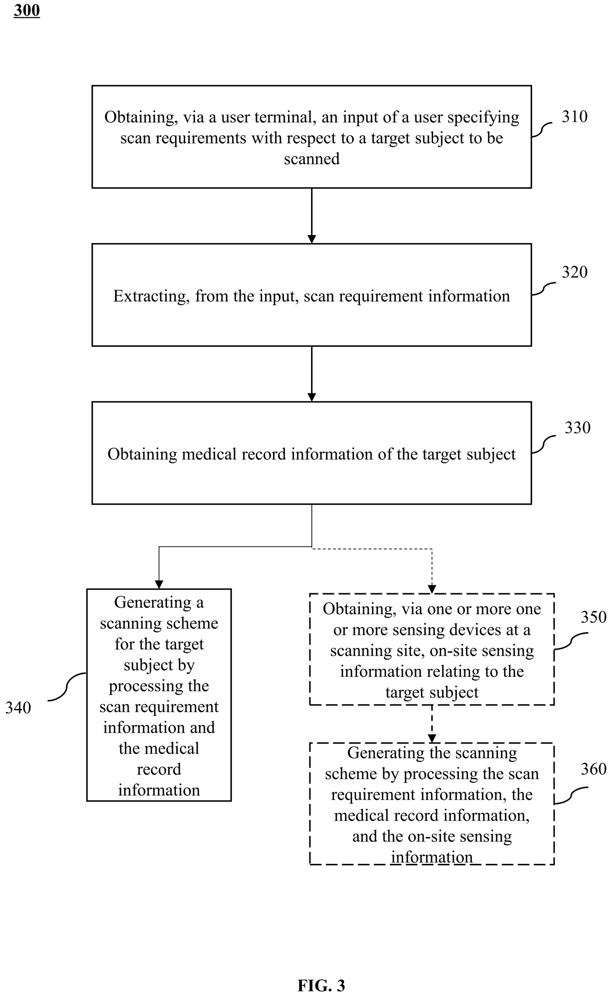

The sensing device 140 refers to a device arranged at a scanning site, configured to collect the on-site sensing information. In some embodiments, the sensing device 140 may include one or more biometric sensors, one or more environmental sensors, one or more device status sensors, etc. The one or more biometric sensors are configured to collect physiological information of the target subject. The one or more environmental sensors are configured to collect environmental information of the scanning site. The one or more device status sensors are configured to collect operational status information of a medical scanning device. Correspondingly, the on-site sensing information may include the physiological information, the environmental information, the operational status information, etc. More descriptions regarding the on-site sensing information may be found in FIG. 3 and its related descriptions.

The interaction module 110-2 refers to a module configured to implement an interaction between the intelligent engine 110 and a user. The user refers to an operator who configures a scanning scheme, such as a scanning technician, etc. In some embodiments, the interaction module 110-2 is built based on a natural language processing (NLP) technology and a large language model (LLM), configured to understand and process a user input. In some embodiments, the interaction module 110-2 may be communicatively connected with the user terminal 120, receive the user input from the user terminal 120, process the user input through the natural language processing technology and the large language model, determine feedback information, and send the feedback information to the user through the user terminal 120.

In some embodiments, the interaction module 110-2 is a user intention recognition module based on an NLP network model combined with an LLM, configured to understand the user input to identify a category of user intention and utilize the user intention in a corresponding processing flow. For example, the interaction module 110-2 sends the identified category of user intention to the analysis module 110-4 for further deliberation on a scanning scheme, or to the user terminal for the user to supplement further information, or the like.

In some embodiments, the interaction module 110-2 may process the user input and context information, and output response information, the category of user intention (e.g., a scanning instruction, professional knowledge consultation, a parameter query, etc.) generated by the LLM model, and a confidence score of the category of user (e.g., represented by a numerical value within a range of 0 to 1).

In some embodiments, a specific internal processing flow of the interaction module 110-2 is as follows: for general cases, the category of user intention is quickly identified from the user input through NLP; for complex cases (e.g., the user input is overly complex), the NLP is unable to process the input or the confidence score of the identified category of user intention is low, the LLM is used to understand the user intention, and finally the LLM generates a response.

For example, if the user input is "Arrange a cardiac CTA for this 65-year-old male patient with chest pain", a first phase of processing by the interaction module 110-2 is as follows: the NLP model (e.g., a small BERT model) is used to rapidly (at a millisecond level) implement user intention classification and entity extraction, identify the category of user intention as SCAN _REQUEST (a scan request) with a confidence score of 99%, extract a plurality of entities including {Age: 65, Symptom: chest pain, Examination: cardiac CTA}, and output a structured instruction work order {"action": "generate _scan_plan", "params": {...}}. If the user input is overly complex (e.g., the scan requirement is special and fall into a non-predefined scenario), a second phase of processing by the interaction module 110-2 is activated: the LLM is used for in-depth understanding and generation, contextual information such as an original user query, output information from the NLP, and the patient's medical record information are all sent to the LLM, and the LLM outputs the category of user intention and the corresponding confidence score; finally, a third phase of processing by the interaction module 110-2 is: generating a natural, fluent, and professional text response via the LLM.

The user terminal 120 refers to a terminal device for interacting with the user. For example, the user terminal may include a mobile phone 120-1, a computer 120-3, a tablet 120-2, etc. The user terminal 120 may include a display and an input device. The display may be configured to present information related to the determination of the scanning scheme. The input device may be configured to receive a user input (e.g., a natural language input, a text input, an image input, a gesture input, etc.). For example, the input device includes a microphone, a keyboard, a mouse, a camera, etc.

In some embodiments, the user terminal 120 may present a digital avatar 121 of the intelligent engine 110. The digital avatar 121 of the intelligent engine 110 refers to a virtual representation of the intelligent engine 110, designed to provide the user with a visual interactive experience. The virtual representation may be a 2D or 3D human-like or cartoon-like figure, which supports personalized customization.

In some embodiments, an interactive interface of the user terminal 120 may include a region for displaying monitoring data, a region for displaying real-time patient information, a region for displaying a scan progress and result, a region for displaying a digital avatar (e.g., a digital virtual technician), a user input interaction region, or the like. The digital avatar can be a 2D or 3D virtual digital human.

In some embodiments, the user terminal 120 may be communicatively connected with the interaction module 110-2. For example, the user may input speech through the user terminal 120. The interaction module 110-2 may convert the speech into text, and further extract scan requirement information from the text. The interaction module 110-2 may also send a generated scanning scheme to the user terminal 120 for user confirmation. More descriptions regarding the scan requirement information and the scanning scheme may be found in FIG. 3 and the related descriptions.

The memory module 110-1 refers to a module responsible for storing and managing information related to the decision-making process of the intelligent engine 110.

In some embodiments, the memory module 110-1 may be communicatively connected to the storage device 130, and may retrieve information from the storage device 130 and/or store information into the storage device 130. The storage device 130 is configured to store data and/or instructions. The storage device 130 may include one or more storage components. Each storage component may be an independent device or part of another device. In some embodiments, the storage device 130 may include a random access memory (RAM), a read-only memory (ROM), a mass storage, a removable memory, a volatile read-write memory, or any combination thereof. Merely by way of example, the mass storage may include a magnetic disk, an optical disk, a solid-state disk, etc.

In some embodiments, information in the storage device 130 may include medical record information, long-term memory information, short-term memory information, etc. More descriptions regarding the medical record information, the short-term memory information, and the long-term memory information may be found in FIG. 3 and the related descriptions. In some embodiments, the storage device 130 may store one or more programs and/or instructions to execute for performing exemplary methods described in the present disclosure. For example, the intelligent engine 110 may execute instructions in the storage device 130 to perform a process 300 shown in FIG. 3. In some embodiments, the storage device 130 may be a portion of the intelligent engine 110.

The analysis module 110-4 refers to a module configured to analyze multi-source data and generate the scanning scheme, and is a core decision-making unit of the intelligent engine 110. In some embodiments, the analysis module 110-4 may be communicatively connected to the sensing module 110-3, the interaction module 110-2, and the memory module 110-1, so as to generate the scanning scheme based on information obtained from the sensing module 110-3, the interaction module 110-2, and the memory module 110-1.

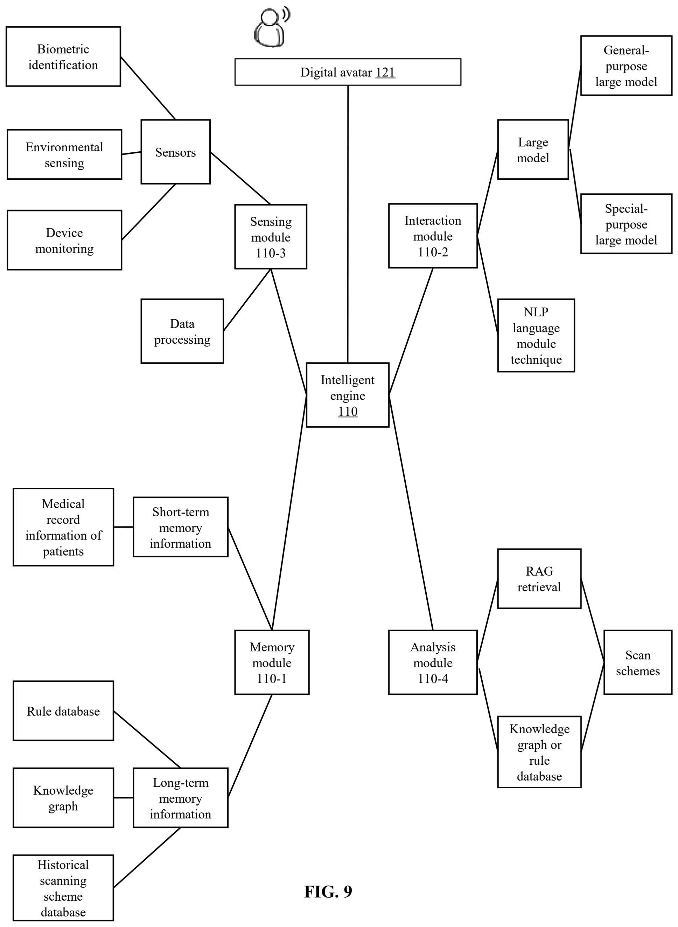

FIG. 9 is a block diagram of an intelligent engine according to some embodiments of the present disclosure. As shown in FIG. 9, the intelligent engine includes the memory module 110-1, the interaction module 110-2, the sensing module 110-3, and the analysis module 110-4. In some embodiments, the sensing module 110-3 may perform biometric identification, environmental sensing, and device monitoring via sensors, and conduct data processing on the obtained information. The memory module 110-1 includes short-term memory information and long-term memory information; the short-term memory information includes medical record information (e.g., basic information), scan reference information, and scan requirement information, while the long-term memory information includes expert experience, historical cases, and operation guidelines. In some embodiments, the short-term memory information includes medical record information of patients. In some embodiments, the long-term memory information includes at least one of a rule database, a knowledge graph, or a historical scanning scheme database. The interaction module 110-2 is built based on a large model and an NLP language technique, the large model may include a general-purpose large model and a special-purpose large model. The analysis module 110-4 may generate scan schemes with reference to the long-term memory information, such as by performing RAG retrieval on the historical scanning scheme database, or querying the knowledge graph or the rule database.

It should be noted that the above descriptions of the medical scanning system 100 are merely for the convenience of descriptions and should not limit the present disclosure to the scope described above. It may be understood that, for those skilled in the art, after understanding the principles of the medical scanning system 100, various devices and modules may be arbitrarily combined, or subsystems may be formed to connect with other devices and modules, without departing from this principle. Those skilled in the art may understand that the medical scanning system 100 shown in FIG. 1 is merely a scenario related to the solution of the present disclosure, and does not constitute a limitation on the modules and computing devices to which the solution of the present disclosure is applied. The sensing module 110-3, the interaction module 110-2, the memory module 110-1, and the analysis module 110-4 may be different modules in one system, or one module may implement functions of two or more of the above modules. A specific computing device may also include more or fewer components than those shown in the figure, or combine some components, or have different component arrangements.

In some embodiments, the medical scanning system 100 may further include a medical scanning device, such as computed tomography (CT), digital subtraction angiography (DSA), magnetic resonance imaging (MRI), or the like. The medical scanning device may be communicatively connected to the intelligent engine 110, and may scan a target subject based on the scanning scheme under control of the intelligent engine 110. Optionally, the intelligent engine 110 may generate a medical image of the target subject based on scan data collected by the medical scanning device.

FIG. 2 is a schematic diagram illustrating a computing device according to some embodiments of the present disclosure. A computing device 200 may be configured to implement the intelligent engine 110 described in FIG. 1.

As shown in FIG. 2, the computing device 200 includes a processor, at least one storage device, a communication interface, a display, and an input device, which are connected via a system bus and an I/O interface. The I/O interface is used for coordination between devices and signal conversion. The processor is configured to provide computing and control capabilities. The at least one storage device includes a non-volatile storage medium and an internal memory. The non-volatile storage medium stores an operating system and a computer program. The internal memory provides an environment for running the operating system and the computer program in the non-volatile storage medium. When the computer program is executed by the processor, the method for medical scanning disclosed in the present disclosure is implemented. The communication interface is configured to perform wired or wireless communication with an external device. The wireless communication may be implemented via WIFI, a mobile cellular network, NFC (Near Field Communication), or other technologies. The display may be a liquid crystal display or an electronic ink display. The input device may be a touch layer covering the display, or may be a key, a trackball, or a touchpad provided on a housing of the computing device 200, or may be an external keyboard, touchpad, or mouse.

Those skilled in the art may understand that the structure shown in FIG. 2 is merely a block diagram of part of the structure related to the solution of the present disclosure, and does not constitute a limitation on the computing device 200 to which the solution of the present disclosure is applied. A specific computing device may include more or fewer components than those shown in the figure, or combine some components, or have different component arrangements.

FIG. 3 is a flowchart illustrating an exemplary process of medical scanning according to some embodiments of the present disclosure. The process 300 may be implemented on a computing device having at least one processor and at least one storage device. For example, the process 300 may be stored in the at least one storage device in the form of instructions, and the process 300 may be implemented when the at least one processor executes the instructions. In some embodiments, the computing device is an intelligent engine (e.g., the intelligent engine 110). For illustration purposes, the implementation of process 300 by the intelligent engine is described below as an example.

In 310, an input of a user is obtained via a user terminal. In some embodiments, the operation 310 may be performed by an interaction module of the intelligent engine.

The input may include a natural language input, an image input, a gesture input, a QR code input, a document input, or a combination of one or more thereof. The natural language input refers to an input provided by the user via the user terminal using a naturally formed language used by humans in daily life. The natural language input may be input via speech or typing.

In some embodiments, the input (e.g., the natural language input) specifies scan requirements for a target subject to be scanned. The target subject to be scanned refers to a subject (e.g., a patient, an animal, a phantom) that needs to undergo scanning. The scan requirements may include identity information of the target subject, which is used to specify the subject to be scanned. The scan requirements may include requirements for a scanning type, a scanning purpose, a scanning site, scanning parameters, image preferences, and may also include other special requirements. In some embodiments, the scan requirements at least include the identity information of the target subject, the scanning purpose, and the scanning site. In some embodiments, the input (e.g., the natural language input) may further include other supplementary information of the target subject, such as a special medical history, etc.

The identity information of the target subject may include information that can characterize the identity of the target subject, such as a medical record number, an ID card number, a name, etc.

The scanning type refers to a classification of technical modalities used in medical scanning. For example, the scanning type may include computed tomography (CT), digital radiography (DR), magnetic resonance imaging (MR), etc., and may be further subdivided, e.g., CT may be subdivided into contrast-enhanced CT, non-contrast CT, CT angiography, perfusion CT, etc. The scanning type may be specified by the user, i.e., the scanning type is included in the input (e.g., the natural language input). Alternatively, the scanning type may not be specified by the user but is determined in the subsequent generation process of the scanning scheme.

The scanning purpose refers to a clinical problem that the scan needs to address, such as tumor screening, pre-operative evaluation, post-operative review, etc. The scanning site may include body portions to be scanned, such as the cervical spine, chest, abdomen, etc. The image preferences refer to requirements for images acquired by the scan, which may include requirements for image quality (e.g., high resolution, high signal-to-noise ratio, etc.), requirements for image features (e.g., sharp image edges, high differentiation between soft and hard tissues, etc.), etc. The special requirements refer to requirements specified based on a personal situation of the target subject, such as using low-dose radiation, not using medication, etc.

For example, the natural language input may be: for the target subject with the medical record number 001, who has undergone a cardiac stent surgery, please recommend a suitable scanning scheme to scan the heart of the target subject for screening of cardiac vascular stenosis.

In some embodiments, the input of the user may be obtained through an input device (e.g., a microphone) of the user terminal. In some embodiments, the input includes the natural language input, a digital avatar of the intelligent engine may be presented through the user terminal, and the natural language input entered by the user during a natural language interaction with the digital avatar may be received. For example, the digital avatar may be a 2D or 3D human figure, cartoon figure, etc., and the digital avatar supports personalized customization. In some embodiments, the intelligent engine may play a speech message through the user terminal, while the digital avatar presented on the user terminal may simulate an expression and gestures of a person speaking, thereby providing the user with a realistic interactive experience. The user describes the scan requirements during the natural language interaction (e.g., a speech conversation) with the digital avatar.

The interaction between the user and the digital avatar may be one or more rounds until the user provides all necessary scan requirements. The interaction may be implemented through the natural language processing technology and the large language model configured on the intelligent engine (e.g., the interaction module). In some embodiments, the natural language input obtained in each of the one or more rounds of the interaction may be processed using the natural language processing technology and the large language model to determine feedback information, and then the feedback information may be played and presented to the user through the user terminal.

In some embodiments of the present disclosure, through the anthropomorphic digital avatar and the natural language interaction, the user does not need to remember complex device parameters and menu paths, and can directly use spoken instructions to complete the scan requirement settings. This interaction manner simplifies the operation process, reduces reliance on the user's professional experience, and enhances the intuitiveness and friendliness of human-computer interaction.

In 320, scan requirement information is extracted from the input. In some embodiments, the operation 320 may be performed by the interaction module of the intelligent engine.

The scan requirement information refers to structured information extracted from the input (e.g., the natural language input), for example, the scan requirement information may be the identity information (e.g., a patient ID), the scanning purpose, the scanning site, the image preferences, the special requirements, etc.

In some embodiments, the intelligent engine may extract the scan requirement information in various ways. For example, if the input (e.g., the natural language input) is in the text form, this input is processed using an NLP model and a LLM model to extract the scan requirement information. If the input (e.g., the natural language input) is in the voice form, this input is converted into text using an Automatic Speech Recognition (ASR) model, and the text is then processed by the NLP model and the LLM model to extract the scan requirement information. If the input is in the image form or document, this input is processed to extract text (e.g., using an optical character recognition technique), and the text is processed using the NLP model and the LLM model to extract the scan requirement information.

In some embodiments, the input (e.g., the natural language input) is provided to a patient registration information system, the patient registration information system pushes a new examination order of the target subject to the intelligent engine, the intelligent engine may extract the scan requirement information and other information from the examination order. Merely by way of example, information in the examination order (such as patient basic information, doctor's scanning requirements, etc.) is extracted to form a structured text input, which is then processed by the NLP model and the LLM model to determine the scan requirement information. In some embodiments, after processing, the scan requirement information is converted into speech via a Text-to-Speech (TTS) model and broadcast to the user.

In 330, medical record information of the target subject is obtained. In some embodiments, the operation 330 may be performed by a memory module of the intelligent engine.

The medical record information refers to historical data generated and recorded during historical medical processes of the target subject. For example, the medical record information may include personal information (or referred to as basic information), a medical record, a hospitalization record, an examination report, a medical order, etc., of the target subject. Exemplary personal information includes the name, the age, the gender, etc.

In some embodiments, the intelligent engine may obtain the medical record information of the target subject in various ways. For example, the intelligent engine (e.g., the memory module) may retrieve the medical record information of the target subject from at least one storage device (e.g., the storage device 130) based on the identity information of the target subject. In some embodiments, the medical record information of the target subject is stored in the storage device as short-term memory information. As another example, the intelligent engine (e.g., the memory module) may directly establish a communication connection with a hospital information system and retrieve the medical record information of the target subject from the hospital information system based on the identity information of the target subject.

In 340, a scanning scheme for the target subject is generated by processing the scan requirement information and the medical record information . In some embodiments, the operation 340 may be performed by an analysis module of the intelligent engine.

The scanning scheme refers to an execution plan configured to guide the medical scanning. In some embodiments, the scanning scheme may include the scanning type and the scanning parameters. More descriptions regarding the scanning type may be found in the above descriptions. The scanning parameters refer to operating parameters used by the medical scanning device during scanning. For example, taking CT as an example, the scanning parameters may include a tube voltage, a tube current, a scan duration, a scan slice thickness, etc.

In some embodiments, the processing of the scan requirement information and the medical record information is performed with reference to long-term memory information stored in the at least one storage device.

The long-term memory information refers to a domain knowledge base that is pre-stored and continuously updated in the at least one storage device, used to support intelligent decision-making. In some embodiments, the long-term memory information may include at least one of a rule database, a knowledge graph, or a historical scanning scheme database.

The rule database refers to a structured database recording rigid medical rules. The medical rigid rules include medical rules specified in operation manuals, scanning guidelines, etc., that need to be complied with. For example, the medical rigid rules may include a prohibition of CT scanning during early pregnancy.

The knowledge graph records medical entities and their interrelationships, and is a semantic, network-structured knowledge base. The medical entities include, but are not limited to, a patient population type entity (e.g., pregnant women, children, obese populations, etc.), a disease type entity (e.g., a lung cancer, a myocarditis, etc.), a scanning site entity, a scanning scheme entity, a scanning parameter entity, etc. In some embodiments, the types of association relationships between medical entities may include a plurality of categories. For example, the association relationships between other entities (e.g., the scanning sites, the patient population type, or the disease type) and the scanning scheme may be of various types such as "must use", "optimally adopt", "prohibit use", and "commonly used"; the association relationships between other entities (e.g., the scanning sites, the patient population type, or the disease type) and the scanning parameter may be of various types such as "need to increase" and "need to decrease". In some embodiments, different types of association relationships may have different weights, for example, a weight of "optimally adopt" is higher than that of "commonly used".

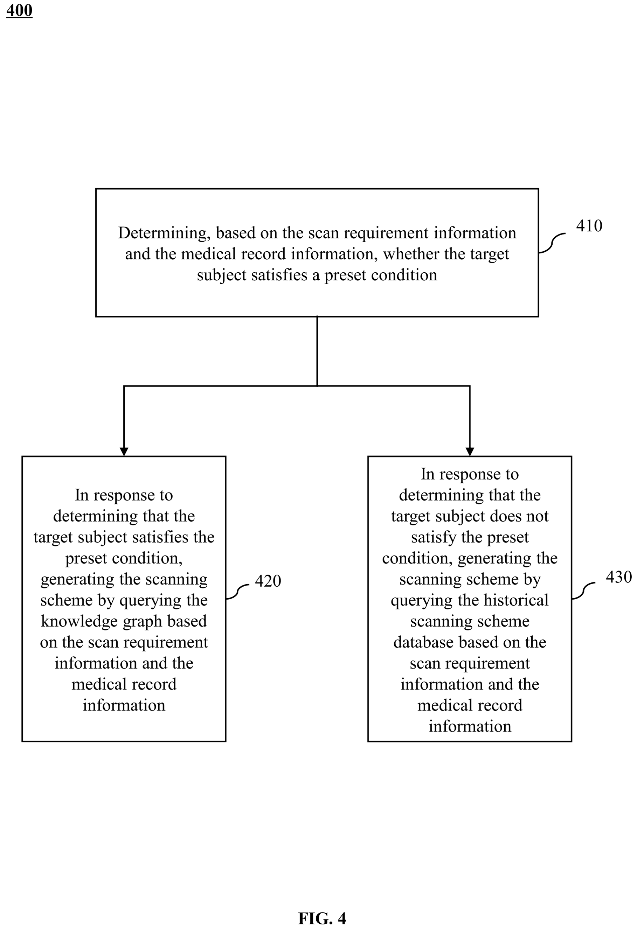

The historical scanning scheme database refers to an experience database recording a plurality of historical scanning schemes and their corresponding basis information. A historical scanning scheme refers to a scanning scheme actually executed in a historical scanning case. The basis information refers to information relied upon when the historical scanning scheme was determined. The basis information includes historical medical record information, historical scan requirement information, historical on-site sensing information, etc., corresponding to a historical scanning subject. The basis information may be stored in a vector form, and may also be referred to as a basis information vector. More descriptions regarding the basis information vector may be found in FIG. 4 and its related descriptions.

In some embodiments, the intelligent engine may generate a scanning scheme for the target subject in a plurality of ways by processing the scan requirement information and the medical record information with reference to the long-term memory information stored in the at least one storage device. For example, the intelligent engine may perform information fusion based on the scan requirement information and the medical record information to form a comprehensive decision rationale. The intelligent engine may match and reason the comprehensive decision rationale with the pre-stored long-term memory information to generate a scanning scheme adapted to the target subject.

In some embodiments, the intelligent engine may determine whether the target subject satisfies a preset condition based on the scan requirement information and the medical record information. In response to determining that the target subject satisfies the preset condition, the intelligent engine may generate the scanning scheme by querying the knowledge graph based on the scan requirement information and the medical record information. In response to determining that the target subject does not satisfy the preset condition, the intelligent engine may generate the scanning scheme by querying the historical scanning scheme database based on the scan requirement information and the medical record information. More descriptions regarding these embodiments may be found in FIG. 4 and the related descriptions.

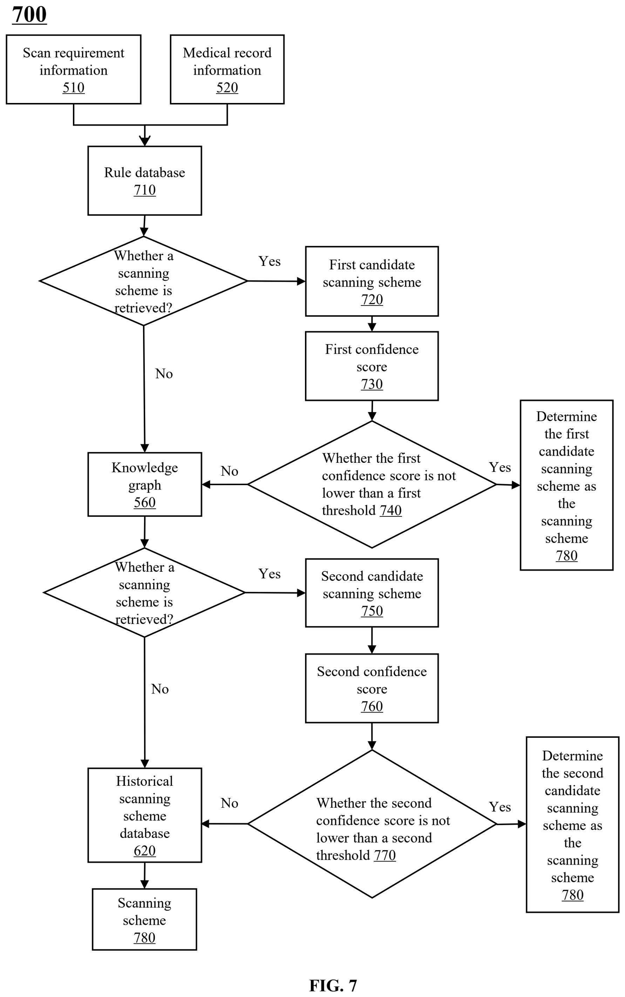

In some embodiments, the intelligent engine may determine a first candidate scanning scheme and its first confidence score by querying the rule database based on the scan requirement information and the medical record information. In response to determining that the first confidence score is lower than a first threshold, the intelligent engine may determine a second candidate scanning scheme and its second confidence score by querying the knowledge graph based on the scan requirement information and the medical record information. In response to determining that the second confidence score is lower than a second threshold, the intelligent engine may determine the scanning scheme by querying the historical scanning scheme database based on the scan requirement information and the medical record information. More descriptions regarding these embodiments may be found in FIG. 7 and the related descriptions.

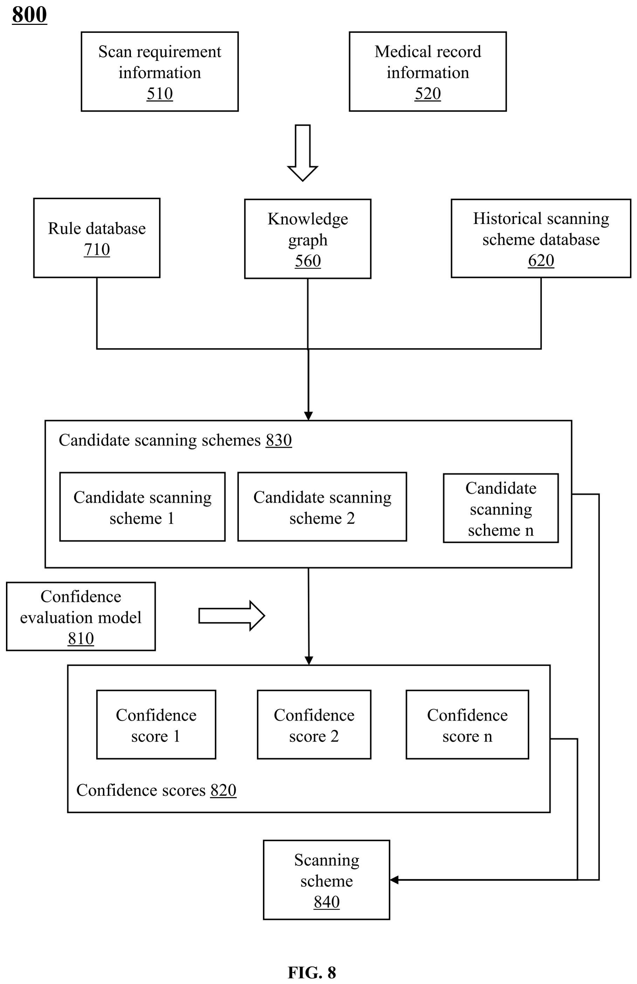

In some embodiments, the intelligent engine may generate at least two candidate scanning schemes by respectively querying the rule database, the knowledge graph, and the historical scanning scheme database based on the scan requirement information and the medical record information. For each of the at least two candidate scanning schemes, the intelligent engine may determine a confidence score of the candidate scanning scheme by processing the scan requirement information, the medical record information, and the candidate scanning scheme using a confidence evaluation model. The intelligent engine may determine the scanning scheme based on the at least two candidate scanning schemes and their confidence scores. More descriptions regarding these embodiments may be found in FIG. 8 and the related descriptions.

In some embodiments, the operation 340 may be omitted, and the intelligent engine may generate the scanning scheme by performing operations 350 and 360.

In 350, on-site sensing information related to the target subject is obtained via one or more sensing devices at a scanning site. In some embodiments, the operation 350 may be performed by a sensing module of the intelligent engine.

The scanning site refers to an environmental space where the target subject is located and used to perform the medical scanning. For example, the scanning site may be a CT scanning room, an MR scanning room, etc. The scanning site is equipped with the one or more sensing devices. More descriptions regarding the one or more sensing devices may be found in FIG. 1 and its related descriptions.

The on-site sensing information refers to information related to the scanning site. For example, the on-site sensing information may include real-time status information related to various factors such as the target subject, the medical scanning device, and the scanning site environment within the scanning site.

In some embodiments, the on-site sensing information includes at least one of physiological information of the target subject collected by one or more biometric sensors, environmental information of the scanning site collected by one or more environmental sensors, or operational status information of the medical scanning device collected by one or more device status sensors. The physiological information may include a heart rate, a body temperature, a blood pressure, a blood oxygen saturation, etc. The environmental information may include a temperature, a humidity, an air quality, a noise level, etc. The operational status information of the medical scanning device may include a device model, a device startup status, an operational status (e.g., a normal status, a fault status, etc.), etc., of the medical scanning device.

In some embodiments of the present disclosure, by integrating the physiological information of the target subject, the environmental information of the scanning site, and the operating status information of the medical imaging equipment, comprehensive data support is provided for the accurate decision-making of the scanning scheme, thereby ensuring the adaptability and reliability of the subsequently determined scanning scheme.

In 360, the scanning scheme is generated by processing the scan requirement information, the medical record information, and the on-site sensing information. In some embodiments, the operation 360 may be performed by the analysis module of the intelligent engine.

The intelligent engine may process the scan requirement information, the medical record information, and the on-site sensing information based on the long-term memory information to generate the scanning scheme. A process of generating the scanning scheme based on the scan requirement information, the medical record information, and the on-site sensing information is similar to the process of generating the scanning scheme based on the scan requirement information and the medical record information as described in operation 340 and below (e.g., FIG. 4-FIG. 8). For ease of description, the following descriptions (e.g., FIG. 4-FIG. 8) are provided with reference to generating the scanning scheme based on the scan requirement information and the medical record information. It should be understood that, unless obviously obtained from the context or the context illustrates otherwise, the term "scan requirement information and medical record information" described below can be replaced by "scan requirement information, medical record information, and on-site sensing information".

In some embodiments of the present disclosure, the collaborative use of multi-source knowledge, such as the rule database, the knowledge graph, the historical scanning scheme database, etc., ensures the compliance, safety, and interpretability of the scanning scheme. Simultaneously, the integration of medical record information, scan requirement information, and on-site sensing information provides a multi-dimensional basis for the decision-making of the scanning scheme, thereby improving the precision and adaptability of the scanning scheme.

In some embodiments, the generated scanning scheme may be presented to the user for confirmation via the user terminal. Optionally, the user may adjust the scanning scheme via the user terminal and confirm the adjusted scanning scheme. After the (adjusted) scanning scheme is confirmed, the target subject may be scanned according to the (adjusted) scanning scheme to acquire scanning data, and a target image of the target subject may be generated based on the scanning data.

In some embodiments, operations 310 and 320 are omitted, and the scanning scheme may be generated based on the medical record information and the on-site sensing information by performing methods for generating the scanning scheme as described in operations 340 and 360.

FIG. 4 is a flowchart illustrating an exemplary process of generating a scanning scheme based on scan requirement information and medical record information according to some embodiments of the present disclosure. A process 400 may be performed to achieve the operation 340 as described in connection with FIG. 4. In some embodiments, the process 400 may be performed by an intelligent engine (e.g., an analysis module).

In 410, whether the target subject satisfies a preset condition is determined based on the scan requirement information and the medical record information.

The preset condition refers to a condition that needs to be satisfied by a patient of a special type. For example, the preset condition may include having a special medical history (e.g., having undergone coronary artery bypass surgery, organ transplant surgery, etc.), belonging to a special population (e.g., children, pregnant women, etc.), or currently presenting an abnormal state (e.g., shock, arrhythmia, etc.). In response to determining that the preset condition is satisfied, the target subject is classified as a special type of patient.

In some embodiments, the intelligent engine may determine whether the target subject satisfies the preset condition based on the scan requirement information and the medical record information in various ways. For example, the intelligent engine (e.g., the analysis module) performs a key feature extraction on the scan requirement information and the medical record information to determine whether the target subject satisfies the preset condition. The key features may be key semantic words included in the preset condition, which may be pre-annotated manually, such as coronary artery bypass surgery, organ transplant surgery, children, pregnant women, shock, arrhythmia, etc. If the key features exist, it is determined that the target subject satisfies the preset condition. The key feature extraction may be implemented based on the natural language processing technology, or the like. In some embodiments, whether the target subject satisfies the preset condition may be further determined based on the on-site sensing information.

In 420, in response to determining that the target subject satisfies the preset condition, the scanning scheme is generated by querying the knowledge graph based on the scan requirement information and the medical record information.

The target subject satisfying the preset condition indicates that the target subject is a patient of the special type, knowledge graph reasoning is required, i.e., a safe and reliable deterministic scanning scheme for the target subject is determined by retrieving a pre-built knowledge graph based on the scan requirement information and the medical record information. More descriptions regarding the knowledge graph may be found in FIG. 3 and its related descriptions.

In some embodiments, in response to determining that the target subject satisfies the preset condition, the intelligent engine may generate the scanning scheme by querying the knowledge graph based on the scan requirement information and the medical record information in various ways. For example, the intelligent engine may extract the key features from the scan requirement information and the medical record information to determine a disease type, determine a scanning scheme associated with the disease type in the knowledge graph by querying the knowledge graph, and determine the scanning scheme as the scanning scheme for the target subject.

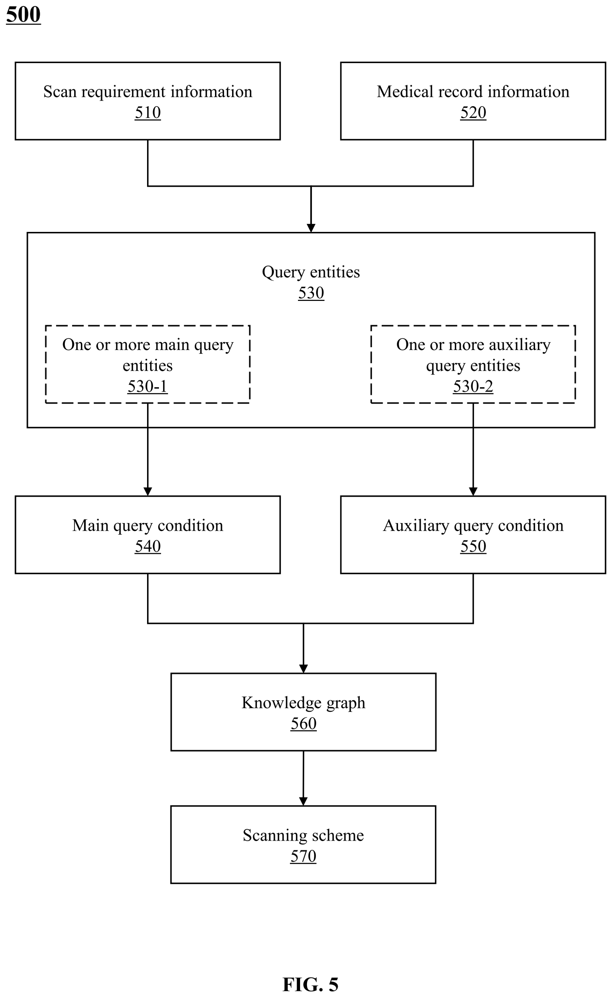

In some embodiments, the intelligent engine may extract query entities from the scan requirement information and the medical record information, the query entities include one or more main query entities and one or more auxiliary query entities. The intelligent engine may construct a main query condition based on the one or more main query entities, construct an auxiliary query condition based on the one or more auxiliary query entities, and generate the scanning scheme by querying the knowledge graph using the main query condition and the auxiliary query condition. More descriptions regarding the embodiment herein may be found in FIG. 5 and its related descriptions.

In 430, in response to determining that the target subject does not satisfy the preset condition, the scanning scheme is generated by querying the historical scanning scheme database based on the scan requirement information and the medical record information.

The target subject not satisfying the preset condition indicates that the target subject is an ordinary patient without special circumstances, and thus a personalized scanning scheme may be generated based on empirical knowledge by querying the historical scanning scheme database. More descriptions regarding the historical scanning scheme database may be found in FIG. 3 and its related descriptions.

In some embodiments, in response to determining that the target subject does not satisfy the preset condition, the intelligent engine may generate the scanning scheme by querying the historical scanning scheme database based on the scan requirement information and the medical record information in various ways. For example, as described above, basis information corresponding to a historical scanning scheme may be stored in the historical scanning scheme database in the form of a basis information vector. The intelligent engine may construct a query vector based on the scan requirement information and the medical record information. The intelligent engine may calculate a similarity (or referred to as a matching degree) between the query vector and each basis information vector in the historical scanning scheme database. The intelligent engine may determine a historical scanning scheme corresponding to a basis information vector with the highest similarity as the scanning scheme for the target subject. Alternatively, the intelligent engine may retrieve historical scanning schemes corresponding to the top N basis information vectors with the highest similarities, and perform a comprehensive analysis on these historical scanning schemes to generate a scanning scheme adapted to the scan requirement information and the medical record information of the target subject.

The basis information vector may be a numerical vector constructed by an embedding model based on historical scan requirement information and historical medical record information (or historical scan requirement information, historical medical record information, and historical on-site sensing information) of a historical scanning scheme. The query vector may be a numerical vector constructed by the embedding model based on the scan requirement information and the medical record information of the target subject. The embedding model refers to a model that converts unstructured data, such as text, images, and audio, into numerical vectors.

In some embodiments, the intelligent engine may query the historical scanning scheme database using a retrieval-augmented generation (RAG) model based on the scan requirement information and the medical record information, so as to generate the scanning scheme.

The retrieval-augmented generation (RAG) refers to a technical framework that combines information retrieval with the generation capability of a large language model.

In some embodiments, a specific application process of the retrieval-augmented generation (RAG) model is as follows: the intelligent engine converts the scan requirement information and the medical record information of the target subject into a query vector through the embedding model. The RAG model may retrieve a historical scanning scheme with the highest matching degree to the query vector from the historical scanning scheme database; and by leveraging the generation and reasoning capabilities of the large language model (LLM), a recommended and personalized scanning scheme is obtained based on the historical scanning scheme with the highest matching degree, which serves as the scanning scheme for the target subject.

In some embodiments of the present disclosure, querying the historical scanning scheme database using the retrieval-augmented generation (RAG) model enables the combination of precise retrieval of historical scanning schemes with the reasoning capability of a large language model. With the RAG model, a personalized scanning scheme tailored to the scan requirement information and medical record information for a non-special type of target subject can be quickly generated. This manner ensures the reliability of the scanning scheme by relying on real historical scanning schemes and achieves flexible adaptation of the scanning scheme through the generation capability of the large language model, thereby improving the generation efficiency and applicability of scanning schemes for ordinary patients.

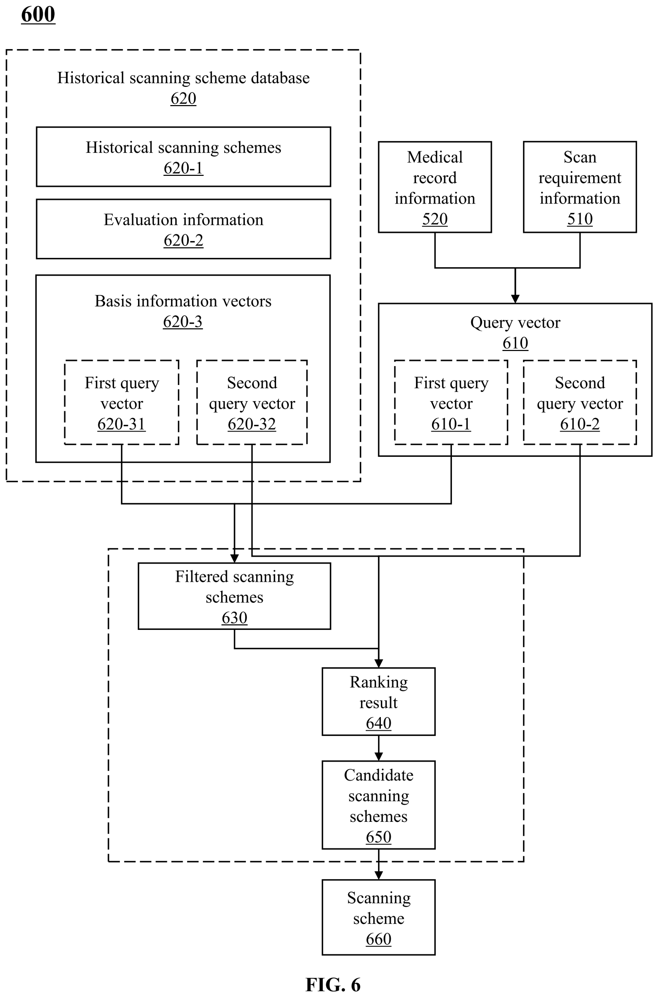

In some embodiments, the intelligent engine may construct the query vector based on the medical record information and the scan requirement information, determine candidate scanning schemes from historical scanning schemes based on the query vector and the basis information vectors of the historical scanning schemes, and determine a candidate scanning scheme with the highest rank among the candidate scanning schemes as the scanning scheme. More descriptions regarding the embodiment herein may be found in FIG. 6 and the related descriptions.

In some embodiments of the present disclosure, for the target subject that satisfies the preset condition, by querying the knowledge graph, a scanning scheme with strong determinism and high safety may be generated, thereby ensuring the standardization and compliance of special case handling. For an ordinary patient that does not satisfy the preset condition, reasoning over the historical scanning scheme database using the RAG model can generate a scanning scheme that leverages historical experience and has a high degree of personalization, thereby achieving efficient and optimized handling of routine cases.

FIG. 5 is a schematic diagram illustrating an exemplary process 500 of generating a scanning scheme by querying a knowledge graph according to some embodiments of the present disclosure. The process 500 may be performed to achieve operation 420 as described in connection with FIG. 4. In some embodiments, the process 500 may be performed by an intelligent engine (e.g., an analysis module).

As shown in FIG. 5, the intelligent engine may extract query entities 530 from the scan requirement information 510 and the medical record information 520, the query entities 530 includes one or more main query entities 530-1 and one or more auxiliary query entities 530-2; the intelligent engine may construct a main query condition 540 based on the one or more main query entities 530-1 and construct an auxiliary query condition 550 based on the one or more auxiliary query entities 530-2; and generate the scanning scheme 570 by querying the knowledge graph 560 using the main query condition 540 and the auxiliary query condition 550.

The query entities refer to key information extracted from the scan requirement information and the medical record information. The query entities may be contained within medical entities in the knowledge graph, for example, the query entities may include a disease type, a scanning site, a patient population type, a scanning purpose, a special medical history, an image preference, a physiological parameter, or the like. In some embodiments, the intelligent engine may perform a structured parsing on the scan requirement information and the medical record information through the natural language processing technology, or the like, to extract the query entities. In some embodiments, the query entities may be further determined based on on-site sensing information.

The one or more main query entities refer to important query entities that have a decisive influence on the scanning scheme (e.g., directly affecting a selection of the scanning type). For example, the one or more main query entities may include a scanning site, a scanning purpose, a patient population type, a disease type, or the like.

In some embodiments, the one or more main query entities are associated with the scanning type. Types of association relationships between the one or more main query entities and the scanning type may include a plurality of relationship types, for example, "must use", "optimally adopt", "prohibit use", and "commonly used". For example, enhanced CT scanning is commonly used for liver space-occupying lesion screening, while high-radiation-dose scanning is prohibited for pregnant women.

The one or more auxiliary query entities refer to secondary query entities that have a relatively minor influence on the scanning scheme (e.g., affecting scanning parameter settings). For example, the one or more auxiliary query entities may include a patient population type, an image preference, a special medical history, a physiological parameter, or the like. In some embodiments, a certain query entity may serve as both a main query entity and an auxiliary query entity, which may affect both the scanning type and scanning parameters. In some embodiments, the one or more main query entities and the one or more auxiliary query entities are determined according to preset entity classification rules.

In some embodiments, the one or more auxiliary query entities are associated with the scanning parameters. Types of association relationships between the one or more auxiliary query entities and a scanning parameter may include a plurality of relationship types, for example, "need to increase the scanning parameter", "need to decrease the scanning parameter", "no need to adjust the scanning parameter", and "prohibit use of high scanning parameter". For example, the tube current needs to be decreased for pediatric patients, and the tube voltage needs to be increased for high signal-to-noise ratio requirements.

The main query condition refers to a core query condition constructed based on the one or more main query entities and used to determine the scanning scheme. The auxiliary query condition refers to an auxiliary query criterion constructed based on the one or more auxiliary query entities and used to optimize the scanning parameters.

Constructing knowledge graph query conditions is a process of converting the query entities into a query language specific to the knowledge graph. The query language includes a SPARQL query language, a GraphQL query language, a natural language query language, or the like. For example, the main query condition may be "perform scanning for [scanning site: liver] to achieve the scanning purpose [detect space-occupying lesions]", and the auxiliary query condition may be "[special medical history: hepatitis B], [patient population type: obese population], [image preference: high signal-to-noise ratio]", or the like.

In some embodiments, the intelligent engine may treat the main query condition and the auxiliary query condition as a parallel constraint set, querying the knowledge graph to retrieve the set of scanning scheme entities that simultaneously satisfy this parallel constraint set. The scanning scheme entity with the highest comprehensive score from this set, or its fine-tuned version, is then determined as the final scanning scheme. The comprehensive score can be determined according to predefined rules, for example, the entity with the highest score may be the scanning scheme entity whose relationship type with the main query entity in the main query condition carries the greatest weight

In some embodiments, the intelligent engine may obtain a candidate scanning scheme by querying the knowledge graph 560 using the main query condition 540; obtain a parameter adjustment suggestion by querying the knowledge graph 560 using the auxiliary query condition 550; and generate the scanning scheme 570 by adjusting the candidate scanning scheme based on the parameter adjustment suggestion.

The candidate scanning scheme refers to a scanning scheme retrieved from the knowledge graph based on the main query condition.

In some embodiments, the intelligent engine may find scanning schemes connected to the one or more main query entities in the main query condition by retrieving the knowledge graph using the main query condition. In some embodiments, each of the one or more main query entities may be used as a starting point to traverse the knowledge graph in parallel to find all scanning schemes associated with each of the one or more main query entities, thereby forming an initial scheme set; subsequently, scheme filtering and ranking are performed based on association relationships between the scanning schemes in the set and the one or more main query entities; and a candidate scanning scheme that best match all constraints in the main query condition is output. For example, when the type of association relationship between a scanning scheme and a main query entity is "prohibit use", the scanning scheme may be filtered out from the initial scheme set. If a plurality of scanning schemes remain after filtering, a scanning scheme with a highest weight is determined as the best matching candidate scanning scheme according to weights of the association relationships (e.g., a weight of "optimally adopt" is higher than a weight of "commonly used").

The parameter adjustment suggestion refers to a modification suggestion for scanning parameters in the candidate scanning scheme, which is obtained from the knowledge graph based on the auxiliary query condition. For example, for an obese patient, the parameter adjustment suggestion may be "increase the tube voltage and the tube current".

In some embodiments, the intelligent engine may query the knowledge graph using the auxiliary query condition to find scanning parameters connected to the one or more auxiliary query entities included in the auxiliary query condition. In some embodiments, each of the one or more auxiliary query entities may be determined as a starting point to traverse the knowledge graph in parallel and identify all scanning parameters associated with each of the one or more auxiliary query entities; further, the scanning parameters included in the candidate scanning scheme may be determined from the associated scanning parameters, and the relationship types between these scanning parameters and the one or more auxiliary query entities (e.g., no need to adjust parameters, need to increase parameters) may be determined; subsequently, the parameter adjustment suggestion for the candidate scanning scheme may be determined based on the relationship types.

In some embodiments, the intelligent engine adjusts the scanning parameters in the candidate scanning scheme based on the parameter adjustment suggestion to generate a final scanning scheme.

In some embodiments of the present disclosure, by first determining the candidate scanning scheme based on the main query condition, and then obtaining the parameter adjustment suggestion through the auxiliary query condition and adjusting the scanning parameters of the candidate scanning scheme, both core adaptability of the scanning scheme and fine-tuning of parameters are ensured, thereby further enhancing the personalization and practicality of the scanning scheme.

In some embodiments of the present disclosure, by splitting the one or more main query entities and the one or more auxiliary query entities, and respectively constructing the main query condition and the auxiliary query condition, the knowledge graph querying becomes more targeted, thereby improving the accuracy and adaptability of scanning scheme generation.

In some embodiments, when the intelligent engine uses the main query condition to retrieve the knowledge graph, a scanning scheme connected to the one or more main query entities in the main query condition may not be found sometimes. At this time, it may be determined that the scanning scheme can not be determined through the knowledge graph, and the scanning scheme may then be determined by querying the historical scanning scheme database. Alternatively, a portion of the one or more main query entities in the main query condition may be removed, and the knowledge graph may be queried again to determine the scanning scheme. In some embodiments, when the intelligent engine uses the auxiliary query condition to retrieve the knowledge graph, a scanning parameter connected to the one or more auxiliary query entities in the auxiliary query condition may not be found sometimes. At this time, the candidate scanning scheme determined based on the main query condition may be directly used as the scanning scheme. Alternatively, a portion of the one or more auxiliary query entities in the auxiliary query condition may be removed, and the knowledge graph may be queried again to determine the parameter adjustment suggestion.

FIG. 6 is a schematic diagram illustrating an exemplary process 600 of generating a scanning scheme by querying a historical scanning scheme database according to some embodiments of the present disclosure. The process 600 may be performed to achieve operation 430 as described in connection with FIG. 4. In some embodiments, the process 600 may be executed by an intelligent engine (e.g., an analysis module).

As shown in FIG. 6, the historical scanning scheme database 620 includes historical scanning schemes 620-1 and their respective basis information vectors 620-3 and evaluation information 620-2. The historical scanning schemes 620-1 are ranked according to their evaluation information 620-2 in the historical scanning scheme database 620. The generating the scanning scheme by querying the historical scanning scheme database 620 may include: constructing a query vector 610 based on the medical record information 520 and the scan requirement information 510; determining candidate scanning schemes 650 from the historical scanning schemes 620-1 based on the query vector 610 and the basis information vectors 620-3 of the historical scanning schemes 620-1; and determining a candidate scanning scheme 650 with the highest rank among the candidate scanning schemes 650 as the scanning scheme 660. More descriptions regarding the historical scanning scheme, the query vector, and the basis information vector may be found in FIG. 4 and the related descriptions. In some embodiments, the query vector 610 may be further constructed based on on-site sensing information.

The evaluation information refers to information for evaluating the quality of the historical scanning schemes. In some embodiments, the evaluation information of a historical scanning scheme includes at least one of quality information of a generated image of a historical scanning scheme, user feedback information of the historical scanning scheme, or a historical usage count of the historical scanning scheme.

The quality information of the generated image of the historical scanning scheme includes quality parameters of the generated image, such as signal-to-noise ratio, contrast, artifact level, etc.

The user feedback information of the historical scanning scheme refers to feedback information provided by a user who executed the historical scanning scheme regarding the historical scanning scheme and/or the generated image of the historical scanning scheme. In some embodiments, after the historical scanning is completed, the intelligent engine may actively pop up an evaluation form on a user terminal, and the user may rate the historical scanning scheme and the generated image of the historical scanning scheme in the evaluation form. Alternatively, the intelligent engine may automatically rate the historical scanning scheme based on user behavior (e.g., whether the user modified the scanning scheme and re-scanned) to obtain the user feedback information.

The historical usage count of the historical scanning scheme refers to a cumulative count of times that the historical scanning scheme has been adopted by users for scanning after being recommended to users in the past.

The historical scanning schemes in the historical scanning scheme database may be ranked based on their evaluation information. A historical scanning scheme with better quality of the generated image, better user feedback, and a higher usage count has a higher ranking. In some embodiments, the intelligent engine may perform a standardization processing (e.g., converting to a standard score of 0-1) on the quality information of the generated image, the historical usage count, and the user feedback information included in the evaluation information, then assign weights to the standardized quality information, historical usage count, and user feedback information according to actual needs, and calculate a confidence score for each historical scanning scheme through a weighted summation. The intelligent engine finally ranks all historical scanning schemes in a descending order of confidence scores to obtain ranked historical scanning schemes.

In some embodiments of the present disclosure, by comprehensively utilizing multi-dimensional evaluation data such as quality information of the generated image, user feedback information, and historical usage count, automatic ranking of the historical scanning schemes in the historical scanning scheme database is achieved. Thus, when determining the scanning scheme based on the historical scanning scheme database subsequently, historically validated and effective historical scanning schemes can be automatically identified and preferentially recommended, thereby effectively improving the reliability and operational efficiency of the generated scanning scheme.

In some embodiments, the intelligent engine may determine candidate scanning schemes based on the query vector 610 and the basis information vectors 620-3 of the historical scanning schemes in a plurality of ways. For example, the intelligent engine may calculate similarities (or referred to as matching degrees) between the query vector and the plurality of basis information vectors, rank the basis information vectors in descending order of the similarities, and take the historical scanning schemes corresponding to the top N basis information vectors as the candidate scanning schemes.

In some embodiments, as shown in FIG. 6, the query vector 610 includes a first query vector 610-1 and a second query vector 610-2, and the basis information vector 620-3 includes a first basis information vector 620-31 and a second basis information vector 620-32. The first query vector 610-1 and the first basis information vector 620-31 relate to the one or more main query entities 530-1, and the second query vector 610-2 and the second basis information vector 620-32 relate to the one or more auxiliary query entities 530-2.

To determine the candidate scanning schemes 650, the historical scanning schemes 620-1 may first be filtered based on the first query vector 610-1 and the first basis information vectors 620-31 of the historical scanning schemes 620-1 to obtain filtered scanning schemes 630. The filtered scanning schemes 630 may then be ranked based on the second query vector 610-2 and the second basis information vectors 620-32 of the historical scanning schemes 620-1. The candidate scanning schemes 650 may be determined from the filtered scanning schemes 630 based on a ranking result 640. More descriptions regarding the query vector and the basis information vector may be found in FIG. 4 and its related descriptions. More descriptions regarding the one or more main query entities and the one or more auxiliary query entities may be found in FIG. 5 and its related descriptions.

The filtered scanning schemes refer to historical scanning schemes in the historical scanning scheme database that match the one or more main query entities. For example, the intelligent engine may select a plurality of first basis information vectors that contain the same elements (i.e., the one or more main query entities) as the first query vector, and take the plurality of historical scanning schemes corresponding to the plurality of first basis information vectors as the filtered scanning schemes. Furthermore, the intelligent engine may determine similarities between the second query vector and the plurality of second basis information vectors corresponding to the filtered scanning schemes, and rank the plurality of filtered scanning schemes in descending order of the similarities to obtain the ranking result of the filtered scanning schemes. The intelligent engine may determine filtered scanning schemes ranked in the top N as candidate scanning schemes based on the ranking result of the filtered scanning schemes.

Merely by way of example, the one or more main query entities include an examination site and a clinical problem, and the one or more auxiliary query entities include a special medical history and an image preference. A batch of historical scanning schemes focusing on the same scanning site and the same clinical problem may be quickly selected as the filtered scanning schemes based on the examination site and the clinical problem. Then, the filtered scanning schemes may be ranked based on the special medical history and the image preference, and the top N filtered scanning schemes with the special medical history and the image preference closer to the current scanning may be selected as the candidate scanning schemes.

In some embodiments of the present disclosure, the filtered scanning schemes matching the historical scanning cases may be quickly selected by using the first query vector, and then the filtered scanning schemes may be ranked by using the second query vector. In this way, the core features (the one or more main query entities) may be used to quickly narrow the retrieval scope and improve the retrieval efficiency, and other auxiliary features (the one or more auxiliary query entities) may be used for accurate ranking, thereby enhancing the scheme adaptability and taking into account both the retrieval speed and the result quality.

In some embodiments, the intelligent engine may directly determine a candidate scanning scheme with the highest ranking among the candidate scanning schemes as the scanning scheme for scanning the target subject.

In some embodiments of the present disclosure, the historical scanning schemes are pre-ranked based on the evaluation information to achieve a comprehensive evaluation of the quality of the historical scanning schemes. The query vector and the basis information vectors are combined to accurately match candidate scanning schemes that satisfy the current scanning requirements. Therefore, through dual screening from two perspectives of requirement adaptability and scheme quality, an optimal scanning scheme is determined from the historical scanning schemes. This process not only improves the generation efficiency of the scanning scheme but also ensures the adaptability and reliability of the scheme.

FIG. 7 is a schematic diagram illustrating another exemplary process 700 of generating a scanning scheme based on scan requirement information and medical record information according to some embodiments of the present disclosure. The process 700 may be performed to achieve operation 340 as described in connection with FIG. 3. In some embodiments, the process 700 may be performed by an intelligent engine (e.g., an analysis module).

As shown in FIG. 7, the intelligent engine may query a rule database 710, the knowledge graph 560, and the historical scanning scheme database 620 sequentially based on the scan requirement information 510 and the medical record information 520 until a scanning scheme that satisfies a condition is determined.

In some embodiments, the rule database 710 may be queried based on the scan requirement information 510 and the medical record information 520, and it may be determined whether a scanning scheme can be retrieved. In response to determining that the scanning scheme can be retrieved from the rule database 710, the retrieved scanning scheme is determined as a first candidate scanning scheme 720, and a first confidence score 730 of the first candidate scanning scheme 720 is determined. It is further determined whether the first confidence score 730 is not lower than a first threshold 740. In response to determining that the first confidence score 730 is not lower than the first threshold 740, the first candidate scanning scheme is determined as the scanning scheme 780. In response to determining that the first confidence score 730 is lower than the first threshold 740, or in response to determining that the scanning scheme can not be retrieved by querying the rule database 710, the knowledge graph 560 is queried based on the scan requirement information 510 and the medical record information 520, and it is determined whether a scanning scheme can be retrieved.

In response to determining that the scanning scheme can be retrieved from the knowledge graph 560, the retrieved scanning scheme is determined as a second candidate scanning scheme 750, and a second confidence score 760 of the second candidate scanning scheme 750 is determined. It is further determined whether the second confidence score 760 is not lower than a second threshold 770. In response to determining that the second confidence score 760 is not lower than the second threshold 770, the second candidate scanning scheme is determined as the scanning scheme 780. In response to determining that the second confidence score 760 is lower than the second threshold 770, or in response to determining that the scanning scheme can not be retrieved by querying the knowledge graph 560, the scanning scheme 780 is determined by querying the historical scanning scheme database 620 based on the scan requirement information 510 and the medical record information 520.

The first candidate scanning scheme 720 refers to a candidate scanning scheme directly derived based on medical rigid rules in the rule database 710.

In some embodiments, the intelligent engine may extract features from the scan requirement information and the medical record information (including a scanning site, a scanning purpose, a special medical history, a disease type, etc.) to form a query feature set, and match the query feature set with rules in the rule database. Feature extraction techniques may include, but are not limited to, the natural language processing techniques, etc. For example, each rule in the rule database corresponds to a standard feature set and a standard scanning scheme. The query feature set may be matched with each standard feature set in the rule database. When the query feature set is completely included in a standard feature set, it is determined that the scanning scheme can be retrieved through the rule database 710, and the standard scanning scheme corresponding to the rule is determined as the first candidate scanning scheme. If the query feature set has mismatched features with all standard feature sets in the rule database, it is determined that the scanning scheme can not be retrieved through the rule database 710.

The first confidence score refers to a numerical indicator used to quantitatively evaluate the reliability of the first candidate scanning scheme. For example, a value of the first confidence score may be in a range of 0 to 1. For example, confidence scores of the rules are pre-stored in the rule database. The confidence score of the rule corresponding to the first candidate scanning scheme may be used as the first confidence score of the first candidate scanning scheme. As another example, the intelligent engine may calculate the first confidence score based on a matching degree between the query feature set and the standard feature set. The higher the matching degree, the higher the first confidence score.

The first threshold refers to a minimum first confidence score required to adopt the first candidate scanning scheme. In some embodiments, the first threshold may be preset manually based on historical experience. In response to determining that the first confidence score of the first candidate scanning scheme is not lower than the first threshold, it indicates that the first candidate scanning scheme has high reliability. Therefore, the intelligent engine may directly determine the first candidate scanning scheme as the scanning scheme without initiating subsequent retrieval and inference processes of the knowledge graph or the historical scanning scheme database. In response to determining that the first confidence score is lower than the first threshold, it indicates that the first candidate scanning scheme does not have high reliability, and the intelligent engine performs a query operation on the knowledge graph.

The second candidate scanning scheme refers to a candidate scanning scheme derived based on the knowledge graph.

In some embodiments, the intelligent engine may perform the knowledge graph query manner described in FIG. 4 or FIG. 5 to retrieve the scanning scheme in the knowledge graph based on the scan requirement information and the medical record information. In response to determining that the scanning scheme can be retrieved, the retrieved scanning scheme is determined as the second candidate scanning scheme.

The second confidence score refers to a numerical indicator used to quantitatively evaluate the reliability of the second candidate scanning scheme. For example, a value of the second candidate scanning scheme may be in a range of 0 to 1. For example, the intelligent engine may directly invoke a pre-stored confidence score corresponding to the second candidate scanning scheme in the knowledge graph and determine the confidence score as the second confidence score. As another example, entities matching the query entities (referred to as matching entities) may be determined when querying the knowledge graph, and the intelligent engine may calculate the second confidence score based on the matching entities.

Merely by way of example, all association relationships between the matching entities in the knowledge graph and a finally determined scanning scheme entity may be determined, and the second confidence score may be determined based on weights of all the association relationships. Different types of association relationships have different weights. A "must use" relationship has the highest weight, an "optimally use" relationship has the next highest weight, and a "commonly use" relationship has a lower weight. The intelligent engine may determine the second confidence score based on a maximum weight of all the association relationships. The greater the maximum weight, the higher the second confidence score.

The second threshold refers to a minimum second confidence score required to adopt the second candidate scanning scheme. In some embodiments, the second threshold may be preset manually based on historical experience. In response to determining that the second confidence score of the second candidate scanning scheme is not lower than the second threshold, it indicates that the second candidate scanning scheme has high reliability. Therefore, the intelligent engine may directly determine the second candidate scanning scheme as the scanning scheme without initiating a subsequent query process for the historical scanning scheme database. If the second confidence score is lower than the second threshold, it indicates that the second candidate scanning scheme does not have high reliability, and the intelligent engine performs a query operation on the historical scanning scheme database.

In some embodiments, the intelligent engine may determine the scanning scheme 780 for the target subject based on the scan requirement information 510 and the medical record information 520 by using the query manner for the historical scanning scheme database described in connection with FIG. 4 or FIG. 6.

Some embodiments of the present disclosure construct a hierarchical progressive retrieval mode by adopting the rule database, the knowledge graph, and the historical scanning scheme database. By configuring confidence thresholds to form a dual screening mechanism, a high degree of adaptation between the scanning scheme and the scan requirement information and the medical record information is ensured. This manner efficiently advances the generation process of the scanning scheme, while enhancing the flexibility and reliability of retrieval, thereby ensuring that an appropriate scanning scheme is able to be output in different scenarios.

In some embodiments, the rule database 710, the knowledge graph 560, and the historical scanning scheme database 620 may be further queried based on the on-site sensing information.

FIG. 8 is another schematic diagram illustrating a process 800 for generating a scanning scheme based on scan requirement information and medical record information according to some embodiments of the present disclosure. The process 800 may be performed to achieve operation 340 as described in connection with FIG. 3. In some embodiments, the process 800 may be performed by an intelligent engine (e.g., an analysis module).

As shown in FIG. 8, the intelligent engine may generate at least two candidate scanning schemes 830 by relatively querying the rule database 710, the knowledge graph 560, and the historical scanning scheme database 620 based on the scan requirement information 510 and the medical record information 520. For each of the at least two candidate scanning schemes 830, a confidence score 820 of the candidate scanning scheme 830 is determined by processing the scan requirement information 510, the medical record information 520, and the candidate scanning scheme 830 using a confidence evaluation model 810. A scanning scheme 840 is determined based on the at least two candidate scanning schemes 830 and their confidence scores 820.

In some embodiments, the intelligent engine may respectively query the rule database, the knowledge graph, and the historical scanning scheme database to obtain the at least two candidate scanning schemes. The at least two candidate scanning schemes may come from at least one of the rule database, the knowledge graph, and the historical scanning scheme database, and each candidate scanning schemes may come from one of the rule database, the knowledge graph, and the historical scanning scheme database. For example, the at least two candidate schemes may include the first candidate scanning scheme 720 obtained by querying the rule database, the second candidate scanning scheme 750 obtained by querying the knowledge graph, and the candidate scanning schemes 650 or the scanning scheme 660 obtained by querying the historical scanning scheme database. More descriptions regarding how to query the rule database, the knowledge graph, and the historical scanning scheme database may be found in FIG. 4 to FIG. 6 and their related descriptions.

A confidence evaluation model refers to a model configured to evaluate a reliability degree (i.e., the confidence score) of a candidate scanning scheme. The confidence evaluation model may be a machine learning model, such as a neural network (NN), or other custom model structures, or any combination thereof.

In some embodiments, an input of the confidence evaluation model may include the scan requirement information, the medical record information, and a candidate scanning scheme, and an output of the confidence evaluation model may include a confidence score of the candidate scanning scheme. In some embodiments, the confidence score may be represented by a value in a range of 0 to 1, a higher confidence score indicates a higher reliability degree of the candidate scanning scheme.

In some embodiments, the confidence evaluation model 810 is a machine learning model trained using training samples and corresponding training labels. The training samples and their corresponding training labels are determined based on the historical scanning scheme database 620. A training sample includes a historical scanning scheme 620-1 and its corresponding basis information (including historical scan requirement information 510 and historical medical record information 520). A training label includes a sample confidence score determined based on evaluation information 620-2 of the historical scanning scheme 620-1.

In some embodiments, the intelligent engine may further construct negative training samples based on scanning guidelines, operation manuals, etc. The intelligent engine uses negative historical scanning schemes (e.g., non-compliant schemes, erroneous schemes) in the historical scanning scheme database and their corresponding basis information (including the historical scan requirement information and the historical medical record information related to the negative historical scanning schemes) as the negative training samples. The intelligent engine sets the sample confidence scores of the negative training samples to 0.

Some embodiments of the present disclosure enable the confidence evaluation model to fully learn the adaptation logic of historical scanning schemes by adopting training samples and labels constructed based on real data in the historical scanning scheme database, thereby ensuring that the evaluation criteria of the confidence evaluation model originate from a large number of real cases verified by practice. Through this manner, the confidence evaluation model is endowed with the ability to learn from successful historical experience, so as to make the output confidence scores of the model possess both objective quantitative characteristics and clinical practice relevance, thereby significantly improving the accuracy and reliability of the evaluation results.

In some embodiments, the input of the confidence evaluation model may further include on-site sensing information. Correspondingly, a training sample may further include historical on-site sensing information corresponding to a historical scanning scheme.

In some embodiments, the confidence evaluation model may be trained based on a large number of training samples with training labels. The intelligent engine may input the training samples into an initial model, determine a value of a loss function based on the training labels and model prediction results, and iteratively update the initial model based on the value of the loss function through manners such as gradient descent. When the loss function satisfies a preset condition, the trained confidence evaluation model is obtained. The preset condition may include convergence of the loss function, a count of iterations reaching a threshold, etc.

In some embodiments, the intelligent engine may determine the scanning scheme (also referred to as a target scanning scheme) in a plurality of ways based on the at least two candidate scanning schemes and their confidence scores. For example, the intelligent engine may determine a candidate scanning scheme with a highest confidence score as the scanning scheme for the target subject.

In some embodiments, the intelligent engine may display the at least two candidate scanning schemes 830, their respective confidence scores 820, and their respective decision rationale via the user terminal 120, receive a user selection input by the user via the user terminal 120, and determine the scanning scheme 840 from the at least two candidate scanning schemes based on the user selection.

The decision rationale characterizes a basis for generating each candidate scanning scheme. For example, the decision rationale may be a type of the long-term memory information relied upon (e.g., the rule database, the knowledge graph, and the historical scanning scheme database), a rule matched in the rule database, matching entities and relationships in the knowledge graph, similar historical scanning schemes and basis information in the historical scanning scheme database, etc.

In some embodiments, the intelligent engine may present the at least two candidate scanning schemes, their respective confidence scores, and decision rationale to the user through the user terminal in various presentation manners. For example, the intelligent engine may rank the at least two candidate scanning schemes in descending order of the numerical values of the confidence scores, simultaneously display the confidence scores of the candidate scanning schemes numerically, and describe their corresponding decision rationale in text. After viewing the presented information through the user terminal, the user may directly select a candidate scanning scheme as a final scanning scheme, or modify the selected candidate scanning scheme and then use the modified candidate scanning scheme as the final scanning scheme.

In some embodiments, after the scanning scheme is determined, the intelligent engine may control the medical scanning device to scan the target subject according to the scanning scheme, and generate a medical image of the target subject based on scan data.

In some embodiments of the present disclosure, by presenting confidence scores and decision rationale of the candidate scanning schemes, more reference information may be provided to the user, so that the user may select the scanning scheme more quickly and accurately.

In some embodiments of the present disclosure, by querying the rule database, the knowledge graph, and the historical scanning scheme database in parallel to generate the at least two candidate scanning schemes, and performing a secondary evaluation using the confidence evaluation model, the reliability of a final output scanning scheme is ensured.

It should be noted that the descriptions of the processes 300-800 are provided for the purposes of illustration, and are not intended to limit the scope of the present disclosure. For persons having ordinary skills in the art, various variations and modifications may be conducted under the teaching of the present disclosure. For example, the processes 300-800 may be accomplished with one or more additional operations not described, and/or without one or more of the operations discussed. Additionally, the order in which the operations of the processes 300-800 are not intended to be limiting. However, those variations and modifications may not depart from the protection of the present disclosure.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of the present disclosure are not necessarily all referring to the same embodiment. In addition, some features, structures, or characteristics of one or more embodiments in the present disclosure may be properly combined.

Furthermore, the recited order of processing elements or sequences, or the use of numbers, letters, or other designations therefore, is not intended to limit the claimed processes and methods to any order except as may be specified in the claims. Although the above disclosure discusses some embodiments of the invention currently considered useful by various examples, it should be understood that such details are for illustrative purposes only, and the additional claims are not limited to the disclosed embodiments. Instead, the claims are intended to cover all combinations of corrections and equivalents consistent with the substance and scope of the embodiments of the invention. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that object of the present disclosure requires more features than the features mentioned in the claims. Rather, claimed subject matter may lie in less than all features of a single foregoing disclosed embodiment.

In some embodiments, the numbers expressing quantities or properties used to describe and claim certain embodiments of the present disclosure are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes. History application documents that are inconsistent or conflictive with the contents of the present disclosure are excluded, as well as documents (currently or subsequently appended to the present specification) limiting the broadest scope of the claims of the present disclosure. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for medical scanning, implemented on a computing device having at least one processor and at least one storage device, the method comprising:
obtaining, via a user terminal, an input of a user specifying scan requirements with respect to a target subject to be scanned;
extracting, from the input, scan requirement information;
obtaining medical record information of the target subject; and
generating a scanning scheme for the target subject by processing the scan requirement information and the medical record information, wherein the processing of the scan requirement information and the medical record information is performed with reference to information stored in the at least one storage device, and the long-term memory information comprises at least one of a rule database, a knowledge graph, or a historical scanning scheme database.

2. The method of claim 1, wherein the generating a scanning scheme for the target subject by processing the scan requirement information and the medical record information comprises:
obtaining, via one or more sensing devices at a scanning site, on-site sensing information relating to the target subject; and
generating the scanning scheme by processing the scan requirement information, the medical record information, and the on-site sensing information.

3. The method of claim 1 or 2, wherein the input includes a natural language input, and the natural language input is obtained by:
presenting, via the user terminal, a digital avatar of an intelligent engine implemented on the at least one processor; and
receiving the natural language input of the user during a natural language interaction with the digital avatar.

4. The method of any one of claims 1 to 3, wherein the processing the scan requirement information and the medical record information comprises:
determining, based on the scan requirement information and the medical record information, whether the target subject satisfies a preset condition; and
in response to determining that the target subject satisfies the preset condition, generating the scanning scheme by querying the knowledge graph based on the scan requirement information and the medical record information.

5. The method of claim 4, wherein the generating the scanning scheme by querying the knowledge graph based on the scan requirement information and the medical record information comprises:
extracting query entities from the scan requirement information and the medical record information, the query entities including one or more main query entities and one or more auxiliary query entities;
constructing a main query condition based on the one or more main query entities and constructing an auxiliary query condition based on the one or more auxiliary query entities, and
generating the scanning scheme by querying the knowledge graph using the main query condition and the auxiliary query condition.

6. The method of any one of claims 1 to 3, wherein the processing the scan requirement information and the medical record information comprises:
determining, based on the scan requirement information and the medical record information, whether the target subject satisfies a preset condition; and
in response to determining that the target subject does not satisfy the preset condition, generating the scanning scheme by querying the historical scanning scheme database based on the scan requirement information and the medical record information.

7. The method of claim 6, wherein the historical scanning scheme database is queried using a retrieval-augmented generation (RAG) model.

8. The method of claim 6 or 7, wherein the historical scanning scheme database includes historical scanning schemes and their respective basis information vectors and evaluation information, the historical scanning schemes are ranked according to their evaluation information in the historical scanning scheme database, and the generating the scanning scheme by querying the historical scanning scheme database comprises:
constructing a query vector based on the medical record information and the scan requirement information;
determining candidate scanning schemes from the historical scanning schemes based on the query vector and the basis information vectors of the historical scanning schemes;
determining a candidate scanning scheme with the highest rank among the candidate scanning schemes as the scanning scheme.

9. The method of any one of claims 1 to 3, wherein the processing the scan requirement information and the medical record information comprises:
determining a first candidate scanning scheme and its first confidence score by querying the rule database based on the scan requirement information and the medical record information;
in response to determining that the first confidence score is lower than a first threshold, determining a second candidate scanning scheme and its second confidence score by querying the knowledge graph based on the scan requirement information and the medical record information;
in response to determining that the second confidence score is lower than a second threshold, determining the scanning scheme by querying the historical scanning scheme database based on the scan requirement information and the medical record information.

10. The method of any one of claims 1 to 3, wherein the processing the scan requirement information and the medical record information comprises:
generating at least two candidate scanning schemes by respectively querying the rule database, the knowledge graph, and the historical scanning scheme database based on the scan requirement information and the medical record information;
for each of the at least two candidate scanning schemes, determining a confidence score of the candidate scanning scheme by processing the scan requirement information, the medical record information, and the candidate scanning scheme using a confidence evaluation model; and
determining the scanning scheme based on the at least two candidate scanning schemes and their confidence scores.

11. The method of claim 10, wherein the determining the scanning scheme based on the at least two candidate scanning schemes and their confidence scores comprises:
displaying, via the user terminal, the at least two candidate scanning schemes, their respective confidence scores, and their respective decision rationale;
receiving, via the user terminal, a user selection input by the user; and
determining the scanning scheme from the at least two candidate scanning schemes based on the user selection.

12. The method of any one of claims 1-11, wherein:
the rule database records rigid medical rules,
the knowledge graph records medical entities and their interrelationships, and the medical entities at least include a scanning scheme entity, and
the historical scanning scheme database recording a plurality of historical scanning schemes and their corresponding basis information.

13. A system, comprising:
a user terminal;
at least one storage device configured to store long-term memory information, the long-term memory information comprises at least one of a rule database, a knowledge graph, or a historical scanning scheme database;
an intelligent engine communicatively communicated with the user terminal and the at least one storage device, configured to:
obtain, via the user terminal, an input of a user specifying scan requirements with respect to a target subject to be scanned;
extract, from the input, scan requirement information;
obtain medical record information of the target subject; and
generate a scanning scheme for the target subject by processing the scan requirement information and the medical record information, wherein the processing of the scan requirement information and the medical record information is performed with reference to the long-term memory information stored in the at least one storage device.

14. The system of claim 13, wherein the system further comprises one or more sensing devices at a scanning site communicatively connected with the intelligent engine, and to process the scan requirement information and the medical record information, the intelligent engine is further configured to:
obtaining, via the one or more sensing devices, on-site sensing information relating to the target subject; and
generating the scanning scheme by processing the scan requirement information, the medical record information, and the on-site sensing information.

15. A non-transitory computer readable medium, comprising a set of instructions for medical scanning, wherein when executed by at least one processor, the set of instructions direct the at least one processor to effectuate the method of any one of claims 1-12.
